# EUROPEAN PATENT APPLICATION

(11) **EP 3 597 190 A1**
(43) Date of publication of application: **22.01.2020**
(21) Application number: 19187747.1
(22) Date of filing: 17.09.2013
(51) Int. Cl.: A61K 31/4184, A61P 31/14

(54) **METHODS FOR TREATING HEPATITIS C**

(30) Priority: 18.09.2012 US 201261702564 P
(62) Divisional of application: 13773463.8
(71) Applicant: AbbVie Inc., North Chicago, IL 60064 (US)
(72) Inventor: NG, Teresa (lok-Chan), Arlington Heights, IL Illinois 60004 (US); PILOT-MATIAS, Tami J., Green Oaks, IL Illinois 60048 (US); KATI, Warren M., Gurnee, IL Illinois 60031 (US); KRISHNAN, Preethi, Gurnee, IL Illinois 60031 (US); MARING, Clarence J., Palatine, IL Illinois 60067 (US); MISTRY, Neeta C., Mundelein, IL Illinois 60060 (US); REISCH, Thomas J., Kenosha, WI Wisconsin 53142 (US); WAGNER, Rolf, Antioch, IL Illinois 60002 (US); LIU, Dachun, Vernon Hills, IL Illinois 60061 (US); PRATT, John K., Kenosha, WI Wisconsin 53142 (US); MATULENKO, Mark A., Libertyville, IL Illinois 60048 (US); KEDDY, Ryan G., Beach Park, IL Illinois 60087 (US)
(74) Representative: Elkington and Fife LLP

(57) **Abstract**

Pan-genotypic HCV inhibitors are described. This invention also relates to methods of using these inhibitors to treat HCV infection.

## Description

### FIELD

The present invention relates to pan-genotypic HCV inhibitors and methods of using the same to treat HCV infection.

### BACKGROUND

Hepatitis C virus (HCV) is an RNA virus belonging to the Hepacivirus genus in the Flaviviridae family. The enveloped HCV virion contains a positive stranded RNA genome encoding all known virus-specific proteins in a single, uninterrupted, open reading frame. The open reading frame comprises approximately 9500 nucleotides and encodes a single large polyprotein of about 3000 amino acids. The polyprotein comprises a core protein, envelope proteins E1 and E2, a membrane bound protein p7, and the non-structural proteins NS2, NS3, NS4A, NS4B, NS5A and NS5B.

HCV infection is associated with progressive liver pathology, including cirrhosis and hepatocellular carcinoma. Chronic hepatitis C may be treated with peginterferon-alpha in combination with ribavirin. Substantial limitations to efficacy and tolerability remain as many users suffer from side effects, and viral elimination from the body is often inadequate. Therefore, there is a need for new drugs to treat HCV infection.

### SUMMARY

It was surprisingly discovered that methyl {(2S,3R)-1-[(2S)-2- {5-[(2R,5R)-1- {3,5-difluoro-4-[4-(4-fluorophenyl)piperidin-1-yl]phenyl}-5-(6-fluoro-2-{(2S)-1-[N-(methoxycarbonyl)-O-methyl-L-threonyl]pyrrolidin-2-yl}-1H-benzimidazol-5-yl)pyrrolidin-2-yl]-6-fluoro-1H-benzimidazol-2-yl}pyrrolidin-1-yl]-3-methoxy-1-oxobutan-2-yl}carbamate (hereinafter "Compound 1") and its pharmaceutically acceptable salts are pan-genotypic HCV inhibitors. These compounds are effective in inhibiting a wide array of HCV genotypes and variants, such as HCV genotype 1, 2, 3, 4, 5, and 6.

Accordingly, a first aspect of the invention features methods for treating HCV. The methods comprise administering an effective amount of Compound 1 or a pharmaceutically acceptable salt thereof to an HCV patient, regardless of the specific HCV genotype(s) that the patient has. Therefore, the patient preferably is not genotyped before the treatment, and the treatment can be initiated without pre-screening the patient for specific HCV genotypes.

In one embodiment of this aspect of the invention, the patient is infected with genotype 2, such as genotype 2a or 2b. In another embodiment of this aspect of the invention, the patient is infected with genotype 3, such as genotype 3a. In another embodiment of this aspect of the invention, the patient is infected with genotype 4, such as genotype 4a. In yet another embodiment of this aspect of the invention, the patient is infected with genotype 5, such as genotype 5a. In still yet embodiment of this aspect of the invention, the patient is infected with genotype 6, such as genotype 6a.

In another embodiment of this aspect of the invention, Compound 1 or the salt thereof is combined or co-administered with another anti-HCV agent. Non-limiting examples of said another anti-HCV agent include HCV polymerase inhibitors, HCV protease inhibitors, other HCV NS5A inhibitors, CD81 inhibitors, cyclophilin inhibitors, or internal ribosome entry site (IRES) inhibitors. In one example, the patient is infected with genotype 2, such as genotype 2a or 2b. In another example, the patient is infected with genotype 3, such as genotype 3a. In another example, the patient is infected with genotype 4, such as genotype 4a. In yet another example, the patient is infected with genotype 5, such as genotype 5a. In still yet another example, the patient is infected with genotype 6, such as genotype 6a.

In yet another embodiment of this aspect of the invention, Compound 1 or the salt thereof is combined or co-administered with an HCV protease inhibitor or an HCV polymerase inhibitor. In one example, the patient is infected with genotype 2, such as genotype 2a or 2b. In another example, the patient is infected with genotype 3, such as genotype 3a. In another example, the patient is infected with genotype 4, such as genotype 4a. In yet another example, the patient is infected with genotype 5, such as genotype 5a. In still yet another example, the patient is infected with genotype 6, such as genotype 6a.

In another embodiment of this aspect of the invention, Compound 1 or the salt thereof is combined or co-administered with an HCV protease inhibitor. In one example, the patient is infected with genotype 2, such as genotype 2a or 2b. In another example, the patient is infected with genotype 3, such as genotype 3a. In another example, the patient is infected with genotype 4, such as genotype 4a. In yet another example, the patient is infected with genotype 5, such as genotype 5a. In still yet another example, the patient is infected with genotype 6, such as genotype 6a.

In another embodiment of this aspect of the invention, Compound 1 or the salt thereof is combined or co-administered with an HCV polymerase inhibitor. In one example, the patient is infected with genotype 2, such as genotype 2a or 2b. In another example, the patient is infected with genotype 3, such as genotype 3a. In another example, the patient is infected with genotype 4, such as genotype 4a. In yet another example, the patient is infected with genotype 5, such as genotype 5a. In still yet another example, the patient is infected with genotype 6, such as genotype 6a.

In another embodiment of this aspect of the invention, Compound 1 or the salt thereof is combined or co-administered with an HCV protease inhibitor and an HCV polymerase inhibitor. In one example, the patient is infected with genotype 2, such as genotype 2a or 2b. In another example, the patient is infected with genotype 3, such as genotype 3a. In another example, the patient is infected with genotype 4, such as genotype 4a. In yet another example, the patient is infected with genotype 5, such as genotype 5a. In still yet another example, the patient is infected with genotype 6, such as genotype 6a.

In this aspect of the invention, as well as each and every embodiment and example described hereunder, the treatment preferably lasts for less than 24 weeks and does not include administration of interferon to said patient. Such a treatment can, for example, comprise administering Compound 1 or a pharmaceutically acceptable salt thereof, together with an HCV protease inhibitor or an HCV polymerase inhibitor or a combination of an HCV protease inhibitor and an HCV polymerase inhibitor, to said patient. For example, the treatment can comprise administering Compound 1 or a pharmaceutically acceptable salt thereof, together with an HCV protease inhibitor, to said patient. For another example, the treatment can comprise administering Compound 1 or a pharmaceutically acceptable salt thereof, together with an HCV polymerase inhibitor, to said patient. For yet another example, the treatment can comprise administering Compound 1 or a pharmaceutically acceptable salt thereof, together with a combination of an HCV protease inhibitor and an HCV polymerase inhibitor, to said patient.

In this aspect of the invention, as well as each and every embodiment and example described hereunder, the treatment preferably lasts for no more than 12 weeks (e.g., the treatment lasts for 8, 9, 10, 11, or 12 weeks; preferably, the treatment lasts for 12 weeks), and does not include administration of interferon to said patient. Such a treatment can, for example, comprise administering Compound 1 or a pharmaceutically acceptable salt thereof, together with an HCV protease inhibitor or an HCV polymerase inhibitor or a combination of an HCV protease inhibitor and an HCV polymerase inhibitor, to said patient. For example, the treatment can comprise administering Compound 1 or a pharmaceutically acceptable salt thereof, together with an HCV protease inhibitor, to said patient. For another example, the treatment can comprise administering Compound 1 or a pharmaceutically acceptable salt thereof, together with an HCV polymerase inhibitor, to said patient. For yet another example, the treatment can comprise administering Compound 1 or a pharmaceutically acceptable salt thereof, together with a combination of an HCV protease inhibitor and an HCV polymerase inhibitor, to said patient.

In this aspect of the invention, as well as each and every embodiment and example described hereunder, the treatment may or may not include administration of ribavirin to said patient; for example, the treatment can include administration of ribavirin to said patient.

In a second aspect, the present invention features methods of treating HCV. The methods comprising administering an effective amount of Compound 1 or a pharmaceutically acceptable salt thereof to an HCV patient, wherein said patient is infected with HCV genotype 2, 3, 4, 5, or 6.

In one embodiment of this aspect of the invention, the patient is infected with genotype 2, such as genotype 2a or 2b. In another embodiment of this aspect of the invention, the patient is infected with genotype 3, such as genotype 3a. In another embodiment of this aspect of the invention, the patient is infected with genotype 4, such as genotype 4a. In yet another embodiment of this aspect of the invention, the patient is infected with genotype 5, such as genotype 5a. In still yet embodiment of this aspect of the invention, the patient is infected with genotype 6, such as genotype 6a.

In another embodiment of this aspect of the invention, Compound 1 or the salt thereof is combined or co-administered with another anti-HCV agent. Non-limiting examples of said another anti-HCV agent include HCV polymerase inhibitors, HCV protease inhibitors, other HCV NS5A inhibitors, CD81 inhibitors, cyclophilin inhibitors, or internal ribosome entry site (IRES) inhibitors. In one example, the patient is infected with genotype 2, such as genotype 2a or 2b. In another example, the patient is infected with genotype 3, such as genotype 3a. In another example, the patient is infected with genotype 4, such as genotype 4a. In yet another example, the patient is infected with genotype 5, such as genotype 5a. In still yet another example, the patient is infected with genotype 6, such as genotype 6a.

In yet another embodiment of this aspect of the invention, Compound 1 or the salt thereof is combined or co-administered with an HCV protease inhibitor or an HCV polymerase inhibitor. In one example, the patient is infected with genotype 2, such as genotype 2a or 2b. In another example, the patient is infected with genotype 3, such as genotype 3a. In another example, the patient is infected with genotype 4, such as genotype 4a. In yet another example, the patient is infected with genotype 5, such as genotype 5a. In still yet another example, the patient is infected with genotype 6, such as genotype 6a.

In another embodiment of this aspect of the invention, Compound 1 or the salt thereof is combined or co-administered with an HCV protease inhibitor. In one example, the patient is infected with genotype 2, such as genotype 2a or 2b. In another example, the patient is infected with genotype 3, such as genotype 3a. In another example, the patient is infected with genotype 4, such as genotype 4a. In yet another example, the patient is infected with genotype 5, such as genotype 5a. In still yet another example, the patient is infected with genotype 6, such as genotype 6a.

In another embodiment of this aspect of the invention, Compound 1 or the salt thereof is combined or co-administered with an HCV polymerase inhibitor. In one example, the patient is infected with genotype 2, such as genotype 2a or 2b. In another example, the patient is infected with genotype 3, such as genotype 3a. In another example, the patient is infected with genotype 4, such as genotype 4a. In yet another example, the patient is infected with genotype 5, such as genotype 5a. In still yet another example, the patient is infected with genotype 6, such as genotype 6a.

In another embodiment of this aspect of the invention, Compound 1 or the salt thereof is combined or co-administered with an HCV protease inhibitor and an HCV polymerase inhibitor. In one example, the patient is infected with genotype 2, such as genotype 2a or 2b. In another example, the patient is infected with genotype 3, such as genotype 3a. In another example, the patient is infected with genotype 4, such as genotype 4a. In yet another example, the patient is infected with genotype 5, such as genotype 5a. In still yet another example, the patient is infected with genotype 6, such as genotype 6a.

In this aspect of the invention, as well as each and every embodiment and example described hereunder, the treatment preferably lasts for less than 24 weeks and does not include administration of interferon to said patient. Such a treatment can, for example, comprise administering Compound 1 or a pharmaceutically acceptable salt thereof, together with an HCV protease inhibitor or an HCV polymerase inhibitor or a combination of an HCV protease inhibitor and an HCV polymerase inhibitor, to said patient. For example, the treatment can comprise administering Compound 1 or a pharmaceutically acceptable salt thereof, together with an HCV protease inhibitor, to said patient. For another example, the treatment can comprise administering Compound 1 or a pharmaceutically acceptable salt thereof, together with an HCV polymerase inhibitor, to said patient. For yet another example, the treatment can comprise administering Compound 1 or a pharmaceutically acceptable salt thereof, together with a combination of an HCV protease inhibitor and an HCV polymerase inhibitor, to said patient.

In this aspect of the invention, as well as each and every embodiment and example described hereunder, the treatment preferably lasts for no more than 12 weeks (e.g., the treatment lasts for 8, 9, 10, 11, or 12 weeks; preferably, the treatment lasts for 12 weeks), and does not include administration of interferon to said patient. Such a the treatment can, for example, comprise administering Compound 1 or a pharmaceutically acceptable salt thereof, together with an HCV protease inhibitor or an HCV polymerase inhibitor or a combination of an HCV protease inhibitor and an HCV polymerase inhibitor, to said patient. For example, the treatment can comprise administering Compound 1 or a pharmaceutically acceptable salt thereof, together with an HCV protease inhibitor, to said patient. For another example, the treatment can comprise administering Compound 1 or a pharmaceutically acceptable salt thereof, together with an HCV polymerase inhibitor, to said patient. For yet another example, the treatment can comprise administering Compound 1 or a pharmaceutically acceptable salt thereof, together with a combination of an HCV protease inhibitor and an HCV polymerase inhibitor, to said patient.

In this aspect of the invention, as well as each and every embodiment and example described hereunder, the treatment may or may not include administration of ribavirin to said patient; for example, the treatment includes administration of ribavirin to said patient.

The present invention also features Compound 1 or a pharmaceutically acceptable salt thereof for use to treat an HCV patient regardless of the specific HCV genotype(s) that the patient has. Such uses are illustrated in the first aspect of the invention described above, including each and every embodiment and example described thereunder.

The present invention further features Compound 1 or a pharmaceutically acceptable salt thereof for use to treat an HCV patient infected with HCV genotype 2, 3, 4, 5, or 6. Such uses are illustrated in the second aspect of the invention described above, including each and every embodiment and example described thereunder.

Other features, objects, and advantages of the present invention are apparent in the detailed description that follows. It should be understood, however, that the detailed description, while indicating preferred embodiments of the invention, are given by way of illustration only, not limitation. Various changes and modifications within the scope of the invention will become apparent to those skilled in the art from the detailed description.

### DETAILED DESCRIPTION

Compound 1, also known as methyl {(2S,3R)-1-[(2S)-2-{5-[(2R,5R)-1-{3,5-difluoro-4-[4-(4-fluorophenyl)piperidin-1-yl]phenyl}-5-(6-fluoro-2-{(2S)-1-[N-(methoxycarbonyl)-O-methyl-L-threonyl]pyrrolidin-2-yl}-1H-benzimidazol-5-yl)pyrrolidin-2-yl]-6-fluoro-1H-benzimidazol-2-yl}pyrrolidin-1-yl]-3-methoxy-1-oxobutan-2-yl}carbamate, is described in U.S. Patent Application Publication No. 2012/0004196, the entire content of which is incorporated herein by reference.

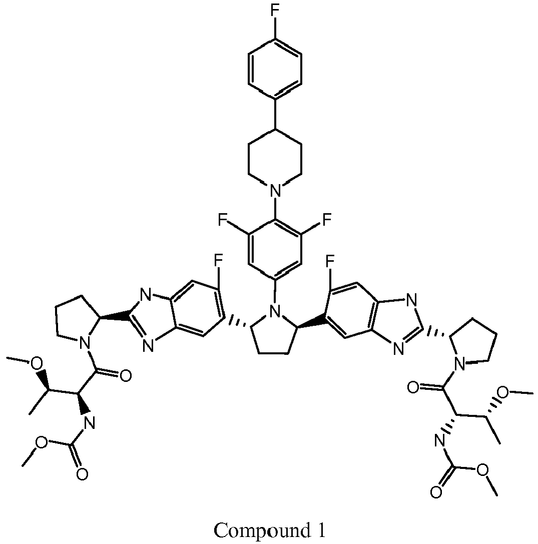

Compound 1 was found to have EC₅₀ values of less than 10 pM against stable subgenomic replicons with NS5A from a broad range of clinically relevant HCV genotypes, such as HCV genotype 1a, 1b, 2a, 2b, 3a, 4a, 5a, and 6a. In transient subgenomic replicon assays, Compound 1 was found to have EC₅₀ values of less than 5 pM against many HCV variants that are resistant to other NS5A inhibitors, such as genotype 2a T24A variant, genotype 2b L28F and L31V variants, genotype 3a M28T and Y93H variants, genotype 4a L28V and L30H variants, genotype 5a L28I, L31F and L31V variants, and genotype 6a L31V, T58N and T58A variants. The EC₅₀ values were determined in the presence of 5% fetal bovine serum but in the absence of human plasma according to the procedures described below.

The present invention features the use of Compound 1 or a pharmaceutically acceptable salt thereof to treat HCV as described hereinabove. In any method or use described herein, Compound 1 or a pharmaceutically acceptable salt thereof can be formulated in a suitable liquid or solid dosage form. Preferably, Compound 1 or the salt thereof is formulated in a solid composition comprising Compound 1 (or a pharmaceutically acceptable salt thereof) in amorphous form, a pharmaceutically acceptable hydrophilic polymer, and optionally a pharmaceutically acceptable surfactant.

A non-limiting way to form an amorphous form of Compound 1 (or a pharmaceutically acceptable salt thereof) is through the formation of solid dispersions with a polymeric carrier. As used herein, the term "solid dispersion" defines a system in a solid state (as opposed to a liquid or gaseous state) comprising at least two components, wherein one component is dispersed throughout the other component or components. For example, an active ingredient or a combination of active ingredients can be dispersed in a matrix comprised of a pharmaceutically acceptable hydrophilic polymer(s) and a pharmaceutically acceptable surfactant(s). The term "solid dispersion" encompasses systems having small particles of one phase dispersed in another phase. These particles are often of less than 400 µm in size, such as less than 100, 10, or 1 µm in size. When a solid dispersion of the components is such that the system is chemically and physically uniform or homogenous throughout or consists of one phase (as defined in thermodynamics), such a solid dispersion is called a "solid solution." A glassy solution is a solid solution in which a solute is dissolved in a glassy solvent.

Any method described herein can employ a solid composition which comprises (1) Compound 1 (or a pharmaceutically acceptable salt thereof) in amorphous form, (2) a pharmaceutically acceptable hydrophilic polymer, and (3) a pharmaceutically acceptable surfactant. Compound 1 (or the salt thereof) and the polymer preferably are formulated in a solid dispersion. The surfactant may also be formulated in the same solid dispersion; or the surfactant can be separately combined or mixed with the solid dispersion.

The hydrophilic polymer can, for example and without limitation, have a T_{g} of at least 50 °C, more preferably at least 60 °C, and highly preferably at least 80 °C including, but not limited to from, 80 °C to 180 °C, or from 100 °C to 150 °C. Preferably, the hydrophilic polymer is water-soluble. Non-limiting examples of suitable hydrophilic polymers include, but are not limited to, homopolymers or copolymers of N-vinyl lactams, such as homopolymers or copolymers of N-vinyl pyrrolidone (e.g., polyvinylpyrrolidone (PVP), or copolymers of N-vinyl pyrrolidone and vinyl acetate or vinyl propionate); cellulose esters or cellulose ethers, such as alkylcelluloses (e.g., methylcellulose or ethylcellulose), hydroxyalkylcelluloses (e.g., hydroxypropylcellulose), hydroxyalkylalkylcelluloses (e.g., hydroxypropylmethylcellulose), and cellulose phthalates or succinates (e.g., cellulose acetate phthalate and hydroxypropylmethylcellulose phthalate, hydroxypropylmethylcellulose succinate, or hydroxypropylmethylcellulose acetate succinate); high molecular polyalkylene oxides, such as polyethylene oxide, polypropylene oxide, and copolymers of ethylene oxide and propylene oxide; polyacrylates or polymethacrylates, such as methacrylic acid/ethyl acrylate copolymers, methacrylic acid/methyl methacrylate copolymers, butyl methacrylate/2-dimethylaminoethyl methacrylate copolymers, poly(hydroxyalkyl acrylates), and poly(hydroxyalkyl methacrylates); polyacrylamides; vinyl acetate polymers, such as copolymers of vinyl acetate and crotonic acid, and partially hydrolyzed polyvinyl acetate (also referred to as partially saponified "polyvinyl alcohol"); polyvinyl alcohol; oligo- or polysaccharides, such as carrageenans, galactomannans, and xanthan gum; polyhydroxyalkylacrylates; polyhydroxyalkyl-methacrylates; copolymers of methyl methacrylate and acrylic acid; polyethylene glycols (PEGs); or any mixture thereof.

Non-limiting examples of preferred hydrophilic polymers include polyvinylpyrrolidone (PVP) K17, PVP K25, PVP K30, PVP K90, hydroxypropyl methylcellulose (HPMC) E3, HPMC E5, HPMC E6, HPMC E15, HPMC K3, HPMC A4, HPMC A15, HPMC acetate succinate (AS) LF, HPMC AS MF, HPMC AS HF, HPMC AS LG, HPMC AS MG, HPMC AS HG, HPMC phthalate (P) 50, HPMC P 55, Ethocel 4, Ethocel 7, Ethocel 10, Ethocel 14, Ethocel 20, copovidone (vinylpyrrolidone-vinyl acetate copolymer 60/40), polyvinyl acetate, methacrylate/methacrylic acid copolymer (Eudragit) L100-55, Eudragit L100, Eudragit S100, polyethylene glycol (PEG) 400, PEG 600, PEG 1450, PEG 3350, PEG 4000, PEG 6000, PEG 8000, poloxamer 124, poloxamer 188, poloxamer 237, poloxamer 338, and poloxamer 407.

Of these, homopolymers or copolymers of N-vinyl pyrrolidone, such as copolymers of N-vinyl pyrrolidone and vinyl acetate, are preferred. A non-limiting example of a preferred polymer is a copolymer of 60 % by weight of N-vinyl pyrrolidone and 40 % by weight of vinyl acetate. Other preferred polymers include, without limitation, hydroxypropyl methylcellulose (HPMC, also known as hypromellose in USP), such as hydroxypropyl methylcellulose grade E5 (HPMC-E5); and hydroxypropyl methylcellulose acetate succinate (HPMC-AS).

The pharmaceutically acceptable surfactant employed can be a non-ionic surfactant. Preferably, the surfactant has an HLB value of from 2-20. A solid composition employed in the invention can also include a mixture of pharmaceutically acceptable surfactants, with at least one surfactant having an HLB value of at least 10 and at least another surfactant having an HLB value of below 10.

Non-limiting examples of suitable pharmaceutically acceptable surfactants include polyoxyethylene castor oil derivates, e.g. polyoxyethyleneglycerol triricinoleate or polyoxyl 35 castor oil (Cremophor® EL; BASF Corp.) or polyoxyethyleneglycerol oxystearate such as polyethylenglycol 40 hydrogenated castor oil (Cremophor® RH 40, also known as polyoxyl 40 hydrogenated castor oil or macrogolglycerol hydroxystearate) or polyethylenglycol 60 hydrogenated castor oil (Cremophor® RH 60); or a mono fatty acid ester of polyoxyethylene sorbitan, such as a mono fatty acid ester of polyoxyethylene (20) sorbitan, e.g. polyoxyethylene (20) sorbitan monooleate (Tween® 80), polyoxyethylene (20) sorbitan monostearate (Tween® 60), polyoxyethylene (20) sorbitan monopalmitate (Tween® 40), or polyoxyethylene (20) sorbitan monolaurate (Tween® 20). Other non-limiting examples of suitable surfactants include polyoxyethylene alkyl ethers, e.g. polyoxyethylene (3) lauryl ether, polyoxyethylene (5) cetyl ether, polyoxyethylene (2) stearyl ether, polyoxyethylene (5) stearyl ether; polyoxyethylene alkylaryl ethers, e.g. polyoxyethylene (2) nonylphenyl ether, polyoxyethylene (3) nonylphenyl ether, polyoxyethylene (4) nonylphenyl ether, polyoxyethylene (3) octylphenyl ether; polyethylene glycol fatty acid esters, e.g. PEG-200 monolaurate, PEG-200 dilaurate, PEG-300 dilaurate, PEG-400 dilaurate, PEG-300 distearate, PEG-300 dioleate; alkylene glycol fatty acid mono esters, e.g. propylene glycol monolaurate (Lauroglycol®); sucrose fatty acid esters, e.g. sucrose monostearate, sucrose distearate, sucrose monolaurate, sucrose dilaurate; sorbitan fatty acid mono esters such as sorbitan mono laurate (Span® 20), sorbitan monooleate, sorbitan monopalnitate (Span® 40), or sorbitan stearate. Other suitable surfactants include, but are not limited to, block copolymers of ethylene oxide and propylene oxide, also known as polyoxyethylene polyoxypropylene block copolymers or polyoxyethylene polypropyleneglycol, such as Poloxamer® 124, Poloxamer® 188, Poloxamer® 237, Poloxamer® 388, or Poloxamer® 407 (BASF Wyandotte Corp.). As described above, a mixture of surfactants can be used in a solid composition employed in the invention.

Non-limiting examples of preferred surfactants include polysorbate 20, polysorbate 40, polysorbate 60, polysorbate 80, Cremophor RH 40, Cremophor EL, Gelucire 44/14, Gelucire 50/13, D-alpha-tocopheryl polyethylene glycol 1000 succinate (vitamin E TPGS), propylene glycol laurate, sodium lauryl sulfate, and sorbitan monolaurate.

The solid dispersion employed in this invention preferably is a solid solution, and more preferably a glassy solution.

In one embodiment, a solid composition employed in the invention comprises an amorphous solid dispersion or solid solution which includes Compound 1 (or a pharmaceutically acceptable salt thereof) and a pharmaceutically acceptable hydrophilic polymer. The solid composition also includes a pharmaceutically acceptable surfactant which preferably is formulated in the amorphous solid dispersion or solid solution. The hydrophilic polymer can be selected, for example, from the group consisting of homopolymer of N-vinyl lactam, copolymer of N-vinyl lactam, cellulose ester, cellulose ether, polyalkylene oxide, polyacrylate, polymethacrylate, polyacrylamide, polyvinyl alcohol, vinyl acetate polymer, oligosaccharide, and polysaccharide. As a non-limiting example, the hydrophilic polymer is selected from the group consisting of homopolymer of N-vinyl pyrrolidone, copolymer of N-vinyl pyrrolidone, copolymer of N-vinyl pyrrolidone and vinyl acetate, copolymer of N-vinyl pyrrolidone and vinyl propionate, polyvinylpyrrolidone, methylcellulose, ethylcellulose, hydroxyalkylcelluloses, hydroxypropylcellulose, hydroxyalkylalkylcellulose, hydroxypropylmethylcellulose, cellulose phthalate, cellulose succinate, cellulose acetate phthalate, hydroxypropylmethylcellulose phthalate, hydroxypropylmethylcellulose succinate, hydroxypropylmethylcellulose acetate succinate, polyethylene oxide, polypropylene oxide, copolymer of ethylene oxide and propylene oxide, methacrylic acid/ethyl acrylate copolymer, methacrylic acid/methyl methacrylate copolymer, butyl methacrylate/2-dimethylaminoethyl methacrylate copolymer, poly(hydroxyalkyl acrylate), poly(hydroxyalkyl methacrylate), copolymer of vinyl acetate and crotonic acid, partially hydrolyzed polyvinyl acetate, carrageenan, galactomannan, and xanthan gum. Preferably, the hydrophilic polymer is selected from polyvinylpyrrolidone (PVP) K17, PVP K25, PVP K30, PVP K90, hydroxypropyl methylcellulose (HPMC) E3, HPMC E5, HPMC E6, HPMC E15, HPMC K3, HPMC A4, HPMC A15, HPMC acetate succinate (AS) LF, HPMC AS MF, HPMC AS HF, HPMC AS LG, HPMC AS MG, HPMC AS HG, HPMC phthalate (P) 50, HPMC P 55, Ethocel 4, Ethocel 7, Ethocel 10, Ethocel 14, Ethocel 20, copovidone (vinylpyrrolidone-vinyl acetate copolymer 60/40), polyvinyl acetate, methacrylate/methacrylic acid copolymer (Eudragit) L100-55, Eudragit L100, Eudragit S100, polyethylene glycol (PEG) 400, PEG 600, PEG 1450, PEG 3350, PEG 4000, PEG 6000, PEG 8000, poloxamer 124, poloxamer 188, poloxamer 237, poloxamer 338, or poloxamer 407. More preferably, the hydrophilic polymer is selected from homopolymers of vinylpyrrolidone (e.g., PVP with Fikentscher K values of from 12 to 100, or PVP with Fikentscher K values of from 17 to 30), or copolymers of 30 to 70% by weight of N-vinylpyrrolidone (VP) and 70 to 30% by weight of vinyl acetate (VA) (e.g., a copolymer of 60% by weight VP and 40% by weight VA). The surfactant can be selected, for example, from the group consisting of polyoxyethyleneglycerol triricinoleate or polyoxyl 35 castor oil (Cremophor® EL; BASF Corp.) or polyoxyethyleneglycerol oxystearate, mono fatty acid ester of polyoxyethylene sorbitan, polyoxyethylene alkyl ether, polyoxyethylene alkylaryl ether, polyethylene glycol fatty acid ester, alkylene glycol fatty acid mono ester, sucrose fatty acid ester, and sorbitan fatty acid mono ester. As a non-limited example, the surfactant is selected from the group consisting of polyethylenglycol 40 hydrogenated castor oil (Cremophor® RH 40, also known as polyoxyl 40 hydrogenated castor oil or macrogolglycerol hydroxystearate), polyethylenglycol 60 hydrogenated castor oil (Cremophor® RH 60), a mono fatty acid ester of polyoxyethylene (20) sorbitan (e.g. polyoxyethylene (20) sorbitan monooleate (Tween® 80), polyoxyethylene (20) sorbitan monostearate (Tween® 60), polyoxyethylene (20) sorbitan monopalmitate (Tween® 40), or polyoxyethylene (20) sorbitan monolaurate (Tween® 20)), polyoxyethylene (3) lauryl ether, polyoxyethylene (5) cetyl ether, polyoxyethylene (2) stearyl ether, polyoxyethylene (5) stearyl ether, polyoxyethylene (2) nonylphenyl ether, polyoxyethylene (3) nonylphenyl ether, polyoxyethylene (4) nonylphenyl ether, polyoxyethylene (3) octylphenyl ether, PEG-200 monolaurate, PEG-200 dilaurate, PEG-300 dilaurate, PEG-400 dilaurate, PEG-300 distearate, PEG-300 dioleate, propylene glycol monolaurate, sucrose monostearate, sucrose distearate, sucrose monolaurate, sucrose dilaurate, sorbitan monolaurate, sorbitan monooleate, sorbitan monopalnitate, and sorbitan stearate. Preferably, the surfactant is selected from polysorbate 20, polysorbate 40, polysorbate 60, polysorbate 80, Cremophor RH 40, Cremophor EL, Gelucire 44/14, Gelucire 50/13, D-alpha-tocopheryl polyethylene glycol 1000 succinate (vitamin E TPGS), propylene glycol laurate, sodium lauryl sulfate, or sorbitan monolaurate. More preferably, the surfactant is selected from sorbitan monolaurate or D-alpha-tocopheryl polyethylene glycol 1000 succinate.

A solid dispersion employed in the invention preferably comprises or consists of a single-phase (defined in thermodynamics) in which Compound 1, or a combination of Compound 1 and another anti-HCV agent, is molecularly dispersed in a matrix containing the pharmaceutically acceptable hydrophilic polymer(s). In such cases, thermal analysis of the solid dispersion using differential scanning calorimetry (DSC) typically shows only one single T_{g}, and the solid dispersion does not contain any detectable crystalline Compound 1 as measured by X-ray powder diffraction spectroscopy.

A solid composition employed in the invention can be prepared by a variety of techniques such as, without limitation, melt-extrusion, spray-drying, co-precipitation, freeze drying, or other solvent evaporation techniques, with melt-extrusion and spray-drying being preferred. The melt-extrusion process typically comprises the steps of preparing a melt which includes the active ingredient(s), the hydrophilic polymer(s) and preferably the surfactant(s), and then cooling the melt until it solidifies. "Melting" means a transition into a liquid or rubbery state in which it is possible for one component to get embedded, preferably homogeneously embedded, in the other component or components. In many cases, the polymer component(s) will melt and the other components including the active ingredient(s) and surfactant(s) will dissolve in the melt thereby forming a solution. Melting usually involves heating above the softening point of the polymer(s). The preparation of the melt can take place in a variety of ways. The mixing of the components can take place before, during or after the formation of the melt. For example, the components can be mixed first and then melted or be simultaneously mixed and melted. The melt can also be homogenized in order to disperse the active ingredient(s) efficiently. In addition, it may be convenient first to melt the polymer(s) and then to mix in and homogenize the active ingredient(s). In one example, all materials except surfactant(s) are blended and fed into an extruder, while the surfactant(s) is molten externally and pumped in during extrusion.

To start a melt-extrusion process, the active ingredient(s) (e.g., Compound 1, or a combination of Compound 1 and at least another anti-HCV agent) can be employed in their solid forms, such as their respective crystalline forms. The active ingredient(s) can also be employed as a solution or dispersion in a suitable liquid solvent such as alcohols, aliphatic hydrocarbons, esters or, in some cases, liquid carbon dioxide. The solvent can be removed, e.g. evaporated, upon preparation of the melt.

Various additives can also be included in the melt, for example, flow regulators (e.g., colloidal silica), binders, lubricants, fillers, disintegrants, plasticizers, colorants, or stabilizers (e.g., antioxidants, light stabilizers, radical scavengers, and stabilizers against microbial attack).

The melting and/or mixing can take place in an apparatus customary for this purpose. Particularly suitable ones are extruders or kneaders. Suitable extruders include single screw extruders, intermeshing screw extruders or multiscrew extruders, preferably twin screw extruders, which can be corotating or counterrotating and, optionally, be equipped with kneading disks. It will be appreciated that the working temperatures will be determined by the kind of extruder or the kind of configuration within the extruder that is used. Part of the energy needed to melt, mix and dissolve the components in the extruder can be provided by heating elements. However, the friction and shearing of the material in the extruder may also provide a substantial amount of energy to the mixture and aid in the formation of a homogeneous melt of the components.

The melt can range from thin to pasty to viscous. Shaping of the extrudate can be conveniently carried out by a calender with two counter-rotating rollers with mutually matching depressions on their surface. The extrudate can be cooled and allow to solidify. The extrudate can also be cut into pieces, either before (hot-cut) or after solidification (cold-cut).

The solidified extrusion product can be further milled, ground or otherwise reduced to granules. The solidified extrudate, as well as each granule produced, comprises a solid dispersion, preferably a solid solution, of the active ingredient(s) in a matrix comprised of the hydrophilic polymer(s) and optionally the pharmaceutically acceptable surfactant(s). Where the granules do not contain any surfactant, a pharmaceutically acceptable surfactant described above can be added to and blended with the granules. The extrusion product can also be blended with other active ingredient(s) and/or additive(s) before being milled or ground to granules. The granules can be further processed into suitable solid oral dosage forms.

The approach of solvent evaporation, via spray-drying, provides the advantage of allowing for processability at lower temperatures, if needed, and allows for other modifications to the process in order to further improve powder properties. The spray-dried powder can then be formulated further, if needed, and final drug product is flexible with regards to whether capsule, tablet or any other solid dosage form is desired.

Exemplary spray-drying processes and spray-drying equipment are described in K. Masters, SPRAY DRYING HANDBOOK (Halstead Press, New York, 4th ed., 1985). Non-limiting examples of spray-drying devices that are suitable for the present invention include spray dryers manufactured by Niro Inc. or GEA Process Engineering Inc., Buchi Labortechnik AG, and Spray Drying Systems, Inc. A spray-drying process generally involves breaking up a liquid mixture into small droplets and rapidly removing solvent from the droplets in a container (spray drying apparatus) where there is a strong driving force for evaporation of solvent from the droplets. Atomization techniques include, for example, two-fluid or pressure nozzles, or rotary atomizers. The strong driving force for solvent evaporation can be provided, for example, by maintaining the partial pressure of solvent in the spray drying apparatus well below the vapor pressure of the solvent at the temperatures of the drying droplets. This may be accomplished by either (1) maintaining the pressure in the spray drying apparatus at a partial vacuum; (2) mixing the liquid droplets with a warm drying gas (e.g., heated nitrogen); or (3) both.

The temperature and flow rate of the drying gas, as well as the spray dryer design, can be selected so that the droplets are dry enough by the time they reach the wall of the apparatus. This help to ensure that the dried droplets are essentially solid and can form a fine powder and do not stick to the apparatus wall. The spray-dried product can be collected by removing the material manually, pneumatically, mechanically or by other suitable means. The actual length of time to achieve the preferred level of dryness depends on the size of the droplets, the formulation, and spray dryer operation. Following the solidification, the solid powder may stay in the spray drying chamber for additional time (e.g., 5-60 seconds) to further evaporate solvent from the solid powder. The final solvent content in the solid dispersion as it exits the dryer is preferably at a sufficiently low level so as to improve the stability of the final product. For instance, the residual solvent content of the spray-dried powder can be less than 2% by weight. Highly preferably, the residual solvent content is within the limits set forth in the International Conference on Harmonization (ICH) Guidelines. In addition, it may be useful to subject the spray-dried composition to further drying to lower the residual solvent to even lower levels. Methods to further lower solvent levels include, but are not limited to, fluid bed drying, infra-red drying, tumble drying, vacuum drying, and combinations of these and other processes.

Like the solid extrudate described above, the spray dried product contains a solid dispersion, preferably a solid solution, of the active ingredient(s) in a matrix comprised of the hydrophilic polymer(s) and optionally the pharmaceutically acceptable surfactant(s). Where the spray dried product does not contain any surfactant, a pharmaceutically acceptable surfactant described above can be added to and blended with the spray-dried product before further processing.

Before feeding into a spray dryer, the active ingredient(s) (e.g., Compound 1, or a combination of Compound 1 and at least another anti-HCV agent), the hydrophilic polymer(s), as well as other optional active ingredients or excipients such as the pharmaceutically acceptable surfactant(s), can be dissolved in a solvent. Suitable solvents include, but are not limited to, alkanols (e.g., methanol, ethanol, 1-propanol, 2-propanol or mixtures thereof), acetone, acetone/water, alkanol/water mixtures (e.g., ethanol/water mixtures), or combinations thereof. The solution can also be preheated before being fed into the spray dryer.

The solid dispersion produced by melt-extrusion, spray-drying or other techniques can be prepared into any suitable solid oral dosage forms. In one embodiment, the solid dispersion prepared by melt-extrusion, spray-drying or other techniques can be compressed into tablets. The solid dispersion can be either directly compressed, or milled or ground to granules or powders before compression. Compression can be done in a tablet press, such as in a steel die between two moving punches. When a solid composition of the present invention comprises Compound 1 and another anti-HCV agent, it is possible to separately prepare solid dispersions of each individual active ingredient and then blend the optionally milled or ground solid dispersions before compacting. Compound 1 and other active ingredient(s) can also be prepared in the same solid dispersion, optionally milled and/or blended with other additives, and then compressed into tablets.

At least one additive selected from flow regulators, binders, lubricants, fillers, disintegrants, or plasticizers may be used in compressing the solid dispersion. These additives can be mixed with ground or milled solid dispersion before compacting. Various other additives may also be used in preparing a solid composition of the present invention, for example dyes such as azo dyes, organic or inorganic pigments such as aluminium oxide or titanium dioxide, or dyes of natural origin; stabilizers such as antioxidants, light stabilizers, radical scavengers, stabilizers against microbial attack.

In any aspect, embodiment and example described herein, Compound 1 (or a pharmaceutically acceptable salt thereof) can be administered to an HCV patient in combination with another anti-HCV agent. Preferably, such a treatment does not include the use of interferon throughout the treatment regimen. The treatment regimen can last, for example and without limitation, 24, 23, 22, 21, 20, 19, 18, 17, 16, 15, 14, 13, 12, 11, 10, 9 or 8 weeks. Preferably, the treatment regimen last, for example and without limitation, 12 weeks. The treatment regimen may also last less than 12 weeks, such as 11, 10, 9 or 8 weeks.

Suitable anti-HCV agents that can be combined with Compound 1 (or a pharmaceutically acceptable salt thereof) include, but are not limited to, HCV polymerase inhibitors (e.g., nucleoside polymerase inhibitors or non-nucleoside polymerase inhibitors), HCV protease inhibitors, HCV helicase inhibitors, other HCV NS5A inhibitors, HCV entry inhibitors, cyclophilin inhibitors, CD81 inhibitors, internal ribosome entry site inhibitors, or any combination thereof. For instance, said another anti-HCV agent can be an HCV polymerase inhibitor. For another instance, said another anti-HCV agent can be an HCV protease inhibitor.

Said another anti-HCV agent can also include two or more HCV inhibitors. For instance, said another anti-HCV agent can be a combination of an HCV polymerase inhibitor and an HCV protease inhibitor. For another instance, said another anti-HCV agent can be a combination of two different HCV protease inhibitors. For another instance, said another anti-HCV agent can be a combination of two different HCV polymerase inhibitors (e.g., one is a nucleoside or nucleotide polymerase inhibitor and the other is a non-nucleoside polymerase inhibitor; or both are nucleoside or nucleotide polymerase inhibitors; or both are non-nucleoside polymerase inhibitor). In yet another example, said another anti-HCV agent can be a combination of another HCV NS5A inhibitor and an HCV polymerase inhibitor. In yet another example, said another anti-HCV agent can be a combination of another HCV NS5A inhibitor and an HCV protease inhibitor. In still another example, said another anti-HCV agent can be a combination of two other HCV NS5A inhibitors.

Specific examples of anti-HCV agents that are suitable for combination with Compound 1 (or a pharmaceutically acceptable salt thereof) in any aspect, embodiment or example described herein include, but are not limited to, PSI-7977 (Pharmasset/Gilead), PSI-7851 (Pharmasset/Gilead), PSI-938 (Pharmasset/Gilead), PF-00868554, ANA-598, IDX184, IDX102, IDX375, GS-9190, VCH-759, VCH-916, MK-3281, BCX-4678, MK-3281, VBY708, ANA598, GL59728, GL60667, BMS-790052, BMS-791325, BMS-650032, BMS-824393, GS-9132, ACH-1095, AP-H005, A-831 (Arrow Therapeutics), A-689 (Arrow Therapeutics), INX08189 (Inhibitex), AZD2836, telaprevir, boceprevir, ITMN-191 (Intermune/Roche), BI-201335, VBY-376, VX-500 (Vertex), PHX-B, ACH-1625, IDX136, IDX316, VX-813 (Vertex), SCH 900518 (Schering-Plough), TMC-435 (Tibotec), ITMN-191 (Intermune, Roche), MK-7009 (Merck), IDX-PI (Novartis), BI-201335 (Boehringer Ingelheim), R7128 (Roche), MK-3281 (Merck), MK-0608 (Merck), PF-868554 (Pfizer), PF-4878691 (Pfizer), IDX-184 (Novartis), IDX-375, PPI-461 (Presidio), BILB-1941 (Boehringer Ingelheim), GS-9190 (Gilead), BMS-790052 (BMS), CTS-1027 (Conatus), GS-9620 (Gilead), PF-4878691 (Pfizer), RO5303253 (Roche), ALS-2200 (Alios BioPharma/Vertex), ALS-2158 (Alios BioPharma/Vertex), GSK62336805 (GlaxoSmithKline), or any combinations thereof.

Non-limiting examples of HCV protease inhibitors that are suitable for combination with Compound 1 (or a pharmaceutically acceptable salt thereof) in any aspect, embodiment or example described herein include ACH-1095 (Achillion), ACH-1625 (Achillion), ACH-2684 (Achillion), AVL-181 (Avila), AVL-192 (Avila), BI-201335 (Boehringer Ingelheim), BMS-650032 (BMS), boceprevir, danoprevir, GS-9132 (Gilead), GS-9256 (Gilead), GS-9451 (Gilead), IDX-136 (Idenix), IDX-316 (Idenix), IDX-320 (Idenix), MK-5172 (Merck), narlaprevir, PHX-1766 (Phenomix), telaprevir, TMC-435 (Tibotec), vaniprevir, VBY708 (Virobay), VX-500 (Vertex), VX-813 (Vertex), VX-985 (Vertex), or any combination thereof. Non-limiting examples of HCV polymerase inhibitors that are suitable for combination with Compound 1 (or a pharmaceutically acceptable salt thereof) in any aspect, embodiment or example described herein include ANA-598 (Anadys), BI-207127 (Boehringer Ingelheim), BILB-1941 (Boehringer Ingelheim), BMS-791325 (BMS), filibuvir, GL59728 (Glaxo), GL60667 (Glaxo), GS-9669 (Gilead), IDX-375 (Idenix), MK-3281 (Merck), tegobuvir, TMC-647055 (Tibotec), VCH-759 (Vertex & ViraChem), VCH-916 (ViraChem), VX-222 (VCH-222) (Vertex & ViraChem), VX-759 (Vertex), GS-6620 (Gilead), IDX-102 (Idenix), IDX-184 (Idenix), INX-189 (Inhibitex), MK-0608 (Merck), PSI-7977 (Pharmasset/Gilead), PSI-938 (Pharmasset/Gilead), RG7128 (Roche), TMC64912 (Medivir), GSK625433 (GlaxoSmithKline), BCX-4678 (BioCryst), ALS-2200 (Alios BioPharma/Vertex), ALS-2158 (Alios BioPharma/Vertex), or any combination thereof. A polymerase inhibitor may be a nucleotide polymerase inhibitor, such as GS-6620 (Gilead), IDX-102 (Idenix), IDX-184 (Idenix), INX-189 (Inhibitex), MK-0608 (Merck), PSI-7977 (Pharmasset/Gilead), PSI-938 (Pharmasset/Gilead), RG7128 (Roche), TMC64912 (Medivir), ALS-2200 (Alios BioPharma/Vertex), ALS-2158 (Alios BioPharma/Vertex), or any combination therefore. A polymerase inhibitor may also be a non-nucleoside polymerase inhibitor, such as ANA-598 (Anadys), BI-207127 (Boehringer Ingelheim), BILB-1941 (Boehringer Ingelheim), BMS-791325 (BMS), filibuvir, GL59728 (Glaxo), GL60667 (Glaxo), GS-9669 (Gilead), IDX-375 (Idenix), MK-3281 (Merck), tegobuvir, TMC-647055 (Tibotec), VCH-759 (Vertex & ViraChem), VCH-916 (ViraChem), VX-222 (VCH-222) (Vertex & ViraChem), VX-759 (Vertex), or any combination thereof. Non-limiting examples of NS5A inhibitors that are suitable for combination with Compound 1 (or a pharmaceutically acceptable salt thereof) in any aspect, embodiment or example described herein include GSK62336805 (GlaxoSmithKline), ACH-2928 (Achillion), ACH-3102 (Achillion), AZD2836 (Astra-Zeneca), AZD7295 (Astra-Zeneca), BMS-790052 (BMS), BMS-824393 (BMS), EDP-239 (Enanta/Novartis), GS-5885 (Gilead), IDX-719 (Idenix), MK-8742 (Merck), PPI-1301 (Presidio), PPI-461 (Presidio), or any combination thereof. Non-limiting examples of cyclophilin inhibitors that are suitable for combination with Compound 1 (or a pharmaceutically acceptable salt thereof) in any aspect, embodiment or example described herein include alisporovir (Novartis & Debiopharm), NM-811 (Novartis), SCY-635 (Scynexis), or any combination thereof. Non-limiting examples of HCV entry inhibitors that are suitable for combination with Compound 1 (or a pharmaceutically acceptable salt thereof) in any aspect, embodiment or example described herein include ITX-4520 (iTherx), ITX-5061 (iTherx), or a combination thereof.

In any aspect, embodiment or example described herein, Compound 1 (or a pharmaceutically acceptable salt thereof) can be administered, for example and without limitation, concurrently with said anther anti-HCV agent. Compound 1 (or a pharmaceutically acceptable salt thereof) can also be administered, for example and without limitation, sequentially with said another anti-HCV agent. For instance, Compound 1 (or a pharmaceutically acceptable salt thereof) can be administered immediately before or after the administration of said another anti-HCV agent. The frequency of administration may be the same or different. For example, Compound 1 (or a pharmaceutically acceptable salt thereof) and said another anti-HCV agent can be administered once daily. For another example, Compound 1 (or a pharmaceutically acceptable salt thereof) can be administered once daily, and said another anti-HCV agent can be administered twice daily.

In any aspect, embodiment or example described herein, Compound 1 (or a pharmaceutically acceptable salt thereof) can be co-formulated with said another anti-HCV agent in a single dosage form. Non-limiting examples of suitable dosage forms include liquid or solid dosage forms. Preferably, the dosage form is a solid dosage form. More preferably, the dosage form is a solid dosage form in which Compound 1 (or a pharmaceutically acceptable salt thereof) is in amorphous form, or highly preferably molecularly dispersed in a matrix which comprises a pharmaceutically acceptable water-soluble polymer and a pharmaceutically acceptable surfactant. Said another anti-HCV agent can also be in amorphous form, or molecularly dispersed in the same matrix or a different matrix which comprises a pharmaceutically acceptable water-soluble polymer and a pharmaceutically acceptable surfactant. Said another anti-HCV agent can also be formulated in different form(s) (e.g., in a crystalline form).

As a non-limiting alternative, Compound 1 (or a pharmaceutically acceptable salt thereof) and said another anti-HCV agent can be formulated in different dosage forms. For instance, Compound 1 (or a pharmaceutically acceptable salt thereof) and said another anti-HCV agent can be formulated in different respective solid dosage forms.

In any aspect, embodiment or example described herein, Compound 1 or a pharmaceutically acceptable salt thereof may be administered in a suitable amount such as, for example, in doses of from about 0.1 mg/kg to about 200 mg/kg body weight, or from about 0.25 mg/kg to about 100 mg/kg, or from about 0.3 mg/kg to about 30 mg/kg. As another non-limiting example, Compound 1 (or a pharmaceutically acceptable salt thereof) may be administered in a total daily dose amount of from about 5 mg to about 300 mg, or from about 25 mg to about 200 mg, or from about 25 mg to about 50 mg or an amount there between. Single dose compositions may contain such amounts or submultiples thereof to make up the daily dose.

It will be understood, however, that the specific dose level for any particular patient will depend upon a variety of factors including the activity of the specific compound employed, the age, body weight, general health, sex, diet, time of administration, route of administration, rate of excretion, drug combination, and the severity of the disease undergoing therapy. It will also be understood that the total daily dosage of the compounds and compositions to be administered will be decided by the attending physician within the scope of sound medical judgment.

The following table lists non-limiting examples of a combination of Compound 1 (or a pharmaceutically acceptable salt thereof) and another anti-HCV agent that can be used in any aspect, embodiment or example described herein. For each treatment, Compound 1 (or a pharmaceutically acceptable salt thereof) and said another anti-HCV agent can be administered daily to an HCV patient. Each treatment can be interferon-free. Administration of ribavirin can be included in each regimen. However, the present invention contemplates that each treatment regimen can be both interferon- and ribavirin-free. In addition, interferon and/or ribavirin can be included in each treatment regimen if needed. Each treatment regimen may also optionally comprise administering one or more other anti-HCV agents to the patient. The duration of each treatment regimen may last, for example and without limitation, 8-48 weeks, depending on the patient's response. In any given regimen described in Table 1, the drugs can be, for example and without limitation, co-formulated in a single solid dosage form. For instance, all drugs used in a regimen can be co-formulated in amorphous forms or molecularly dispersed in a matrix comprising a pharmaceutically acceptable water-soluble polymer and optionally a pharmaceutically acceptable surfactant; for another instance, Compound 1 is formulated in amorphous form or molecularly dispersed in a matrix comprising a pharmaceutically acceptable water-soluble polymer and optionally a pharmaceutically acceptable surfactant, and the other drug is in crystalline form(s) and combined with amorphous Compound 1 in a single solid dosage form. For yet another instance, Compound 1 is formulated in a different dosage form than that of the other drug.

**Table 1. Non-Limiting Examples of Interferon-free Treatment Regimens (with or without ribavirin)**

| **Regimen** | **Drugs used in the treatment** | |
|---|---|---|
| 1 | Compound 1 (or its salt) | ACH-1095 (Achillion) |
| 2 | Compound 1 (or its salt) | ACH-1625 (Achillion) |
| 3 | Compound 1 (or its salt) | ACH-2684 (Achillion) |
| 4 | Compound 1 (or its salt) | ACH-2928 (Achillion) |
| 5 | Compound 1 (or its salt) | alisporivir (Debio 025; Novartis) |
| 6 | Compound 1 (or its salt) | ALS-2158 |
| 7 | Compound 1 (or its salt) | ALS-2200 |
| 8 | Compound 1 (or its salt) | ANA-598 (setrobuvir, Anadys) |
| 9 | Compound 1 (or its salt) | ANA-773 (Anadys) |
| 10 | Compound 1 (or its salt) | AVL-181 (Avila) |
| 11 | Compound 1 (or its salt) | AVL-192 (Avila) |
| 12 | Compound 1 (or its salt) | AZD2836 (Astra-Zeneca) |
| 13 | Compound 1 (or its salt) | AZD7295 (Astra-Zeneca) |
| 14 | Compound 1 (or its salt) | BCX-4678 (BioCryst) |
| 15 | Compound 1 (or its salt) | BI-201335 (Boehringer Ingelheim) |
| 16 | Compound 1 (or its salt) | BI-207127 (Boehringer Ingelheim) |
| 17 | Compound 1 (or its salt) | BILB-1941 (Boehringer Ingelheim) |
| 18 | Compound 1 (or its salt) | BMS-650032 (BMS) |
| 19 | Compound 1 (or its salt) | BMS-790052 (BMS) |
| 20 | Compound 1 (or its salt) | BMS-791325 (BMS) |
| 21 | Compound 1 (or its salt) | BMS-824393 (BMS) |
| 22 | Compound 1 (or its salt) | boceprevir |
| 23 | Compound 1 (or its salt) | CTS-1027 (Conatus) |
| 24 | Compound 1 (or its salt) | danoprevir |
| 25 | Compound 1 (or its salt) | VX-985 (Vertex) |
| 26 | Compound 1 (or its salt) | filibuvir (PF-00868554, Pfizer) |
| 27 | Compound 1 (or its salt) | GL59728 (Glaxo) |
| 28 | Compound 1 (or its salt) | GL60667 (Glaxo) |
| 29 | Compound 1 (or its salt) | GS-5885 (Gilead) |
| 30 | Compound 1 (or its salt) | GS-6620 (Gilead) |
| 31 | Compound 1 (or its salt) | GS-9132 (Gilead) |
| 32 | Compound 1 (or its salt) | GS-9256 (Gilead) |
| 33 | Compound 1 (or its salt) | GS-9451 (Gilead) |
| 34 | Compound 1 (or its salt) | GS-9620 (Gilead) |
| 35 | Compound 1 (or its salt) | GS-9669 (Gilead) |
| 36 | Compound 1 (or its salt) | GSK62336805 |
| 37 | Compound 1 (or its salt) | GSK625433 (GlaxoSmithKline) |
| 38 | Compound 1 (or its salt) | IDX-102 (Idenix) |
| 39 | Compound 1 (or its salt) | IDX-136 (Idenix) |
| 40 | Compound 1 (or its salt) | IDX-184 (Idenix) |
| 41 | Compound 1 (or its salt) | IDX-316 (Idenix) |
| 42 | Compound 1 (or its salt) | IDX-320 (Idenix) |
| 43 | Compound 1 (or its salt) | IDX-375 (Idenix) |
| 44 | Compound 1 (or its salt) | INX-189 (Inhibitex) |
| 45 | Compound 1 (or its salt) | ITX-4520 (iTherx) |
| 46 | Compound 1 (or its salt) | ITX-5061 (iTherx) |
| 47 | Compound 1 (or its salt) | MK-0608 (Merck) |
| 48 | Compound 1 (or its salt) | MK-3281 (Merck) |
| 45 | Compound 1 (or its salt) | MK-5172 (Merck) |
| 50 | Compound 1 (or its salt) | narlaprevir |
| 52 | Compound 1 (or its salt) | NM-811 (Novartis) |
| 53 | Compound 1 (or its salt) | PF-4878691 (Pfizer) |
| 54 | Compound 1 (or its salt) | PHX-1766 (Phenomix) |
| 55 | Compound 1 (or its salt) | PPI-1301 (Presidio) |
| 56 | Compound 1 (or its salt) | PPI-461 (Presidio--) |
| 57 | Compound 1 (or its salt) | PSI-7977 (Pharmasset/Gilead) |
| 58 | Compound 1 (or its salt) | PSI-938 (Pharmasset/Gilead) |
| 59 | Compound 1 (or its salt) | mericitabine (RG7128; Roche) |
| 60 | Compound 1 (or its salt) | RO5303253 (Roche) |
| 61 | Compound 1 (or its salt) | SCY-635 (/Scynexis/) |
| 62 | Compound 1 (or its salt) | tegobuvir |
| 63 | Compound 1 (or its salt) | telaprevir |
| 64 | Compound 1 (or its salt) | TMC-435 (Tibotec) |
| 65 | Compound 1 (or its salt) | TMC-647055 (Tibotec) |
| 66 | Compound 1 (or its salt) | TMC64912 (Medivir) |
| 67 | Compound 1 (or its salt) | vaniprevir |
| 68 | Compound 1 (or its salt) | VBY708 (Virobay) |
| 69 | Compound 1 (or its salt) | VCH-759 (Vertex & ViraChem) |
| 70 | Compound 1 (or its salt) | VCH-916 (ViraChem) |
| 71 | Compound 1 (or its salt) | VX-222 (VCH-222) (Vertex & ViraChem) |
| 72 | Compound 1 (or its salt) | VX-500 (Vertex) |
| 73 | Compound 1 (or its salt) | VX-759 (Vertex) |
| 74 | Compound 1 (or its salt) | VX-813 (Vertex) |
| 75 | Compound 1 (or its salt) | TMC649128 (Medivir) |
| 76 | Compound 1 (or its salt) | tegobuvir (GS-9190; Gilead) |
| 77 | Compound 1 (or its salt) | GI-5005 (GlobeImmune) |
| 78 | Compound 1 (or its salt) | IMO-2125 (Idera//) |
| 79 | Compound 1 (or its salt) | ITX-5061 (ITheRx) |
| 80 | Compound 1 (or its salt) | miR-122 (Regulus) |
| 81 | Compound 1 (or its salt) | Miravirsen (SPC3649; Santaris) |
| 82 | Compound 1 (or its salt) | ACH-3102 |
| 83 | Compound 1 (or its salt) | EDP-239 |
| 84 | Compound 1 (or its salt) | IDX-719 |
| 85 | Compound 1 (or its salt) | MK-8742 |

It should be understood that the above-described embodiments and the following examples are given by way of illustration, not limitation. Various changes and modifications within the scope of the present invention will become apparent to those skilled in the art from the present description.

### Example 1. Antiviral Activity of Compound 1 in HCV Replicon Cell Culture Assays

The inhibitory activities of Compound 1 can be evaluated using the following protocol. Two genotype 1 stable subgenomic replicon cell lines can be used for compound characterization in cell culture: one derived from genotype la-H77 and the other derived from genotype lb-Conl. The replicon constructs can be bicistronic subgenomic replicons. The genotype la replicon construct contains NS3-NS5B coding region derived from the H77 strain of HCV (la-H77). The replicon also has a firefly luciferase reporter and a neomycin phosphotransferase (Neo) selectable marker. These two coding regions, separated by the FMDV 2a protease, comprise the first cistron of the bicistronic replicon construct, with the second cistron containing the NS3-NS5B coding region with addition of adaptive mutations E1202G, K1691R, K2040R, and S2204I. The lb-Conl replicon construct is identical to the la-H77 replicon, except that the HCV 5' UTR, 3' UTR, and NS3-NS5B coding region are derived from the lb-Conl strain, and the adaptive mutations are K1609E, K1846T, and Y3005C. In addition, the lb-Conl replicon construct contains a poliovirus IRES between the HCV IRES and the luciferase gene. Replicon cell lines can be maintained in Dulbecco's modified Eagles medium (DMEM) containing 10% (v/v) fetal bovine serum (FBS), 100 IU/ml penicillin, 100 mg/ml streptomycin (Invitrogen), and 200 mg/ml G418 (Invitrogen). The inhibitory effects of Compound 1 on HCV replication can be determined by measuring the luciferase reporter activity. For example, replicon-containing cells can be seeded into 96-well plates at a density of 5000 cells per well in 100 µl DMEM containing 5% FBS. The following day Compound 1 can be diluted in dimethyl sulfoxide (DMSO) to generate a 200x stock in a series of eight half-log dilutions. The dilution series can then be further diluted 100-fold in the medium containing 5% FBS. Medium with the inhibitor is added to the overnight cell culture plates already containing 100 µl of DMEM with 5% FBS. The cells can be incubated for three days in the tissue culture incubators after which time 30 µl of Passive Lysis buffer (Promega) can be added to each well, and then the plates are incubated for 30-45 minutes with rocking to lyse the cells. Luciferin solution (100 µl, Promega) can be added to each well, and luciferase activity can be measured with a Victor II luminometer (Perkin-Elmer). The percent inhibition of HCV RNA replication can be calculated for each compound concentration and the EC₅₀ value can be calculated using nonlinear regression curve fitting to the 4-parameter logistic equation and GraphPad Prism 4 software.

The ability of Compound 1 to inhibit NS5A from non-genotype 1 HCV can be evaluated according to the following. A number of stable subgenomic 1b-Con1 replicon cell lines containing a portion of NS5A from genotype 2a, 2b, 3a, 4a, 5a or 6a HCV are created. The replicon construct contains a NotI restriction site upstream of NS5A, and a BlpI restriction site just after NS5A amino acid 214. HCV RNA from infected subjects is isolated (see Middleton et al., J VIROL METHODS 145:137-145 (2007), and Tripathi et al., ANTIVIRAL RES 73:40-49 (2007)), and RT-PCR is conducted on the RNA to generate a DNA fragment encoding HCV NS5A amino acids 1-214. The PCR fragment incorporates NotI and BlpI compatible ends, and this fragment is ligated into a plasmid containing the 1b-Con1 replicon. Stable cell lines containing these chimeric replicons are generated by introducing these constructs into Huh-7 cells. The inhibitory effect of Compound 1 on HCV replication in these replicons can be determined by measuring activity of the luciferase reporter gene as described above.

Using the above-described assays or similar cell-based replicon assays, Compound 1 showed significantly inhibitory activities against replication of HCV replicons with NS5A from genotype 1-6 (Table 2).

**Table 2**

| **HCV Replicon Subtype** | **Mean EC₅₀ ± Std. Dev. (pM)** |
|---|---|
| Genotype 1a-H77 | 1.8 ± 0.9 |
| Genotype 1b-Con1 | 4.3 ± 1.7 |
| Genotype 2a | 2.3 ± 0.7 |
| Genotype 2b | 1.9 ± 0.6 |
| Genotype 3a | 2.1 ± 0.7 |
| Genotype 4a | 1.9 ± 0.6 |
| Genotype 5a | 1.4 ± 0.4 |
| Genotype 6a | 2.8 ± 0.7 |

### Example 2. Antiviral Potency of Compound 1 against HCV Non-genotype 1 Wild Type and Variants as Compared to Other HCV NS5A Inhibitors

Compound 1 was tested against mutants resistant to other NS5A inhibitors, including the reference compound shown in Table 3. Transiently replicating chimeric replicons containing NS5A from genotype 2-6 wild types or replicons containing variants within NS5A were constructed in the 1b-Con1 background. These replicons also contained a firefly luciferase reporter gene. Variants were introduced by site-directed mutagenesis using the Change-IT Multiple Mutation Site Directed Mutagenesis Kit (USB). After the mutagenesis was confirmed by sequence analysis, the plasmids were linearized with ScaI restriction enzyme. The TranscriptAid T7 High Yield Transcription Kit (Fermentas) was used to transcribe the HCV subgenomic RNA from the plasmids. The RNA was transfected *via* electroporation into a Huh-7 derived cell line as described (see Middleton *et al.* and Tripathi *et al., supra*) except that 3 × 10⁶ cells were electroporated with 15 µg of template RNA and the 96 well plate was seeded with 7.5 × 10³ cells per well. Four hours post-transfection, the wells from one plate were harvested for luciferase measurement. This plate provided a measure of the amount of translatable input RNA, and therefore transfection efficiency. To the wells of the remaining plates, a half-log dilution series of the test compound in culture medium (0.5% DMSO final concentration) was added, and plates were incubated at 37°C, 5% CO₂ in a humidified incubator for 4 days. After this period, the media was removed and the plates were washed with 100 µl phosphate-buffered saline per well. Luciferin solution (50 µl, Promega) was added to each well, and luciferase activity was measured with a Victor II luminometer (Perkin-Elmer). The percent inhibition of HCV RNA replication was calculated for each compound concentration and the EC₅₀ value was calculated using nonlinear regression curve fitting to the 4-parameter logistic equation and GraphPad Prism 4 software.

Using the above-described assays or similar cell-based replicon assays, Compound 1 showed significant inhibitory activities against replication of HCV replicons containing non-genotype 1 wild type NS5A as well as NS5A with resistant variants (Table 3).

**Table 3**

| **Genotype** | **Mutant** | **Average EC₅₀ (pM) (Compound 1)** | **Average EC₅₀ (pM) (reference compound*)** |
|---|---|---|---|
| 2a | WT | 1.3 | 3.4 |
| | T24A | 1.3 | 92 |
| 2b | WT | 1.0 | 1.1 |
| | L28F | 1.1 | 39 |
| | L31V | 0.9 | 427 |
| 3a | WT | 1.8 | 7.2 |
| | M28T | 0.8 | 3030 |
| | Y93H | 4.3 | >100,000 |
| 4a | WT | 0.9 | 0.4 |
| | L28V | 0.8 | 5.3 |
| | L30H | 1.0 | 0.8 |
| 5a | WT | 1.1 | 0.9 |
| | L28I | 1.0 | 72 |
| | L31F | 1.9 | 263 |
| | L31V | 0.9 | 221 |
| 6a | WT | 1.4 | 80 |
| | T58N | 2.5 | 8468 |
| | L31V | 1.0 | 5035 |
| | T58A | 1.5 | 1145 |

| | | | |
|---|---|---|---|
| * Reference compound is 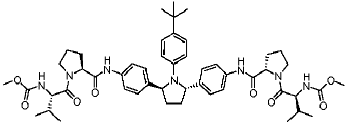 | | | |

The foregoing description of the present invention provides illustration and description, but is not intended to be exhaustive or to limit the invention to the precise one disclosed. Modifications and variations are possible in light of the above teachings or may be acquired from practice of the invention. Thus, it is noted that the scope of the invention is defined by the claims and their equivalents.

The present invention also features the following embodiments:
1. A method of treatment for HCV, comprising administering an effective amount of Compound 1 or a pharmaceutically acceptable salt thereof to an HCV patient, wherein said patient is not genotyped for said treatment.
2. The method of embodiment 1, wherein said patient is infected with HCV genotype 2.
3. The method of embodiment 1, wherein said patient is infected with HCV genotype 3.
4. The method of embodiment 1, wherein said patient is infected with HCV genotype 4.
5. The method of embodiment 1, wherein said patient is infected with HCV genotype 5.
6. The method of embodiment 1, wherein said patient is infected with HCV genotype 6.
7. The method according to one of embodiments 1-6, where said Compound 1 or the salt thereof is co-administered with another anti-HCV agent.
8. The method according to one of embodiments 1-6, wherein said Compound 1 is co-administered with an HCV protease inhibitor or an HCV polymerase inhibitor.
9. The method according to one of embodiments 1-6, wherein said Compound 1 is co-administered with an HCV protease inhibitor and an HCV polymerase inhibitor.
10. The method according to one of embodiments 1-6, wherein said treatment lasts for less than 24 weeks and does not include administration of interferon to said patient.
11. The method according to one of embodiments 1-6, wherein said treatment lasts for no more than 12 weeks and does not include administration of interferon to said patient.
12. The method according to one of embodiments 1-6, wherein said Compound 1 is co-administered with an HCV protease inhibitor or a combination of an HCV protease inhibitor and an HCV polymerase inhibitor, and wherein said treatment lasts for less than 24 weeks and does not include administration of interferon to said patient.
13. The method according to one of embodiments 1-6, wherein said Compound 1 is co-administered with an HCV protease inhibitor or a combination of an HCV protease inhibitor and an HCV polymerase inhibitor, and wherein said treatment lasts for no more than 12 weeks and does not include administration of interferon to said patient.
14. A method of treatment for HCV, comprising administering an effective amount of Compound 1 or a pharmaceutically acceptable salt thereof to an HCV patient, wherein said patient is infected with HCV genotype 2, 3, 4, 5, or 6.
15. The method of embodiment 13, wherein said patient is infected with HCV genotype 2.
16. The method of embodiment 13, wherein said patient is infected with HCV genotype 3.
17. The method of embodiment 13, wherein said patient is infected with HCV genotype 4.
18. The method of embodiment 13, wherein said patient is infected with HCV genotype 5.
19. The method of embodiment 13, wherein said patient is infected with HCV genotype 6.
20. The method according to one of embodiments 14-19, wherein said Compound 1 is co-administered with an HCV protease inhibitor or a combination of an HCV protease inhibitor and an HCV polymerase inhibitor, and wherein said treatment lasts for less than 24 weeks and does not include administration of interferon to said patient.
21. The method according to one of embodiments 14-19, wherein said Compound 1 is co-administered with an HCV protease inhibitor or a combination of an HCV protease inhibitor and an HCV polymerase inhibitor, and wherein said treatment lasts for no more than 12 weeks and does not include administration of interferon to said patient.

## Claims

1. Methyl {(2S,3R)-1-[(2S)-2-{5-[(2R,5R)-1-{3,5-difluoro-4-[4-(4-fluorophenyl)piperidin-1-yl]phenyl}-5-(6-fluoro-2-{(2S)-1-[N-(methoxycarbonyl)-O-methyl-L-threonyl]pyrrolidin-2-yl}-1H-benzimidazol-5-yl)pyrrolidin-2-yl]-6-fluoro-1H-benzimidazol-2-yl}pyrrolidin-1-yl]-3-methoxy-1-oxobutan-2-yl}carbamate (Compound 1) or a pharmaceutically acceptable salt thereof for use in a method of treatment for HCV, comprising administering an effective amount of Compound 1 or a pharmaceutically acceptable salt thereof to an HCV patient in combination with another anti-HCV agent, regardless of the specific HCV genotype(s) that the patient has, wherein said patient is not genotyped for said treatment, and wherein said treatment regimen lasts 24, 23, 22, 21, 20, 19, 18, 17, 16, 15, 14, 13, 12, 11, 10, 9 or 8 weeks.

2. Methyl {(2S,3R)-1-[(2S)-2-{5-[(2R,5R)-1-{3,5-difluoro-4-[4-(4-fluorophenyl)piperidin-1-yl]phenyl}-5-(6-fluoro-2-{(2S)-1-[N-(methoxycarbonyl)-O-methyl-L-threonyl]pyrrolidin-2-yl}-1H-benzimidazol-5-yl)pyrrolidin-2-yl]-6-fluoro-1H-benzimidazol-2-yl}pyrrolidin-1-yl]-3-methoxy-1-oxobutan-2-yl}carbamate (Compound 1) or a pharmaceutically acceptable salt thereof for use in a method of treatment for HCV, comprising administering an effective amount of Compound 1 or a pharmaceutically acceptable salt thereof to an HCV patient in combination with another anti-HCV agent, wherein said patient is infected with HCV genotype 2, 3, 4, 5, or 6, and wherein said treatment regimen lasts 24, 23, 22, 21, 20, 19, 18, 17, 16, 15, 14, 13, 12, 11, 10, 9 or 8 weeks.

3. Compound 1 or a pharmaceutically acceptable salt thereof for use according to claim 1 or 2, wherein said treatment does not include the use of interferon throughout the treatment regimen.

4. Compound 1 or a pharmaceutically acceptable salt thereof for use according to claim 1 or 2, wherein said another anti-HCV agent is an HCV protease inhibitor.

5. Compound 1 or a pharmaceutically acceptable salt thereof for use according to claim 1 or 2, co-formulated with said another anti-HCV agent in a single dosage form.

6. Compound 1 or a pharmaceutically acceptable salt thereof for use according to claim 1 or 2, wherein said treatment regimen lasts 16 weeks.

7. Compound 1 or a pharmaceutically acceptable salt thereof for use according to claim 1 or 2, wherein said treatment regimen lasts 12 weeks.

8. Compound 1 or a pharmaceutically acceptable salt thereof for use according to claim 1 or 2, wherein said treatment regimen lasts less than 12 weeks.

9. Compound 1 or a pharmaceutically acceptable salt thereof for use according to claim 1 or 2, wherein said treatment regimen last 8 weeks.

10. Compound 1 or a pharmaceutically acceptable salt thereof for use according to claim 1 or 2, wherein said patient is infected with HCV genotype 2.

11. Compound 1 or a pharmaceutically acceptable salt thereof for use according to claim 1 or 2, wherein said patient is infected with HCV genotype 3.

12. Compound 1 or a pharmaceutically acceptable salt thereof for use according to claim 1 or 2, wherein said patient is infected with HCV genotype 4.

13. Compound 1 or a pharmaceutically acceptable salt thereof for use according to claim 1 or 2, wherein said patient is infected with HCV genotype 5.

14. Compound 1 or a pharmaceutically acceptable salt thereof for use according to claim 1 or 2, wherein said patient is infected with HCV genotype 6.
